# EUROPEAN PATENT SPECIFICATION

(11) **EP 0 812 847 B1**
(45) Date of publication and mention of the grant of the patent: **25.09.2002**
(21) Application number: 97304114.8
(22) Date of filing: 12.06.1997
(51) Int. Cl.: C07F 5/02, C07C 43/29, C07C 39/27, C07F 3/02

(54) **Process for the preparation of 5-bromo-2-fluorobenzeneboronic acid**
Verfahren zur Herstellung von 5-Brom-2-Fluorbenzolboronsäure
Procédé de préparation de l'acide 5-bromo-2-fluorobenzèneboronique

(30) Priority: 14.06.1996 US 663755
(43) Date of publication of application: 17.12.1997
(73) Proprietor: BASF AKTIENGESELLSCHAFT, 67056 Ludwigshafen (DE)
(72) Inventor: Barnes, Keith Douglas, Newtown, Pennsylvania 18940 (US)
(74) Representative: Langfinger, Klaus Dieter, Dr.

(56) References cited:
- EP-A- 0 238 272
- EP-A- 0 440 082
- DAVID L. LADD: "Improved Synthesis of Fluoroveratroles and Fluorophenethylamines via Organolithium Reagents" JOURNAL OF ORGANIC CHEMISTRY., vol. 46, no. 1, - 1981 EASTON US, pages 203-206, XP002041329
- ANDREW W. FERNHAM: "The Pyrethrins and Related Compounds. Part XXXVII." PESTICIDE SCIENCE., vol. 28, no. 1, - 1990 BARKING GB, pages 25-34, XP002041330

## Description

5-Bromo-2-fluorobenzeneboronic acid is an important intermediate in the synthesis of a number of non-ester pyrethroid compounds. 5-Bromo-2-fluorobenzeneboronic acid and processes for its preparation are described in U.S. 5,068,403 and Pesticide Science, 28, pp. 25-34 (1990). Those references disclose that 5-bromo-2-fluorobenzeneboronic acid is prepared from 2,4-dibromofluorobenzene. However, 2,4-dibromofluorobenzene is not entirely satisfactory for use in the commercial manufacture of 5-bromo-2-fluoro benzeneboronic acid. EP 440082 discloses the synthesis of 2,5-difluorobenzeneboronic acid via reaction of 1,4-difluorobenzene with Bu Li, followed by reaction with B(OMe)₃ at -65°C. Ladd et al (J. Org. Chem, 981, vol.46 No. 1, pages 203-206) give more experimental detail, and discloses a yield of 63%.

2,4-Dibromofluorobenzene is commercially available as a mixture containing seventy percent 2,4-dibromofluoro benzene and thirty percent 3,4-dibromofluorobenzene. When that mixture is used to prepare 5-bromo-2-fluorobenzene boronic acid, at most, only a 70% yield is obtainable based on the total amount used. In addition, to obtain high purity 5-bromo-2-fluorobenzeneboronic acid, a time consuming purification step is required to remove impurities such as 3,4-dibromofluorobenzene. A process that avoids the use of 2,4-dibromofluorobenzene would provide a great improvement over the art processes.

It is therefore an object of the present invention to provide a process for the preparation of 5-bromo-2-fluoro benzeneboronic acid which avoids the use of 2,4-dibromo-fluorobenzene. The present invention provides a process for the preparation of 5-bromo-2-fluorobenzeneboronic acid which comprises lithiating 1-bromo-4-fluorobenzene, which is conveniently carried out with a lithium base in the presence of a solvent, to form (5-bromo-2-fluorophenyl) lithium, reacting (5-bromo-2-fluorophenyl)lithium with a tri(C₁-C₆alkyl) borate to form a di(C₁-C₆ alkyl) 5-bromo-2-fluorobenzeneboronate, and hydrolyzing the di(C₁-C₆ alkyl) 5-bromo-2-fluorobenzeneboronate. It has been found that the process of this invention is more effective and efficient than the prior art processes, and avoids the use of 2,4-dibromo-fluorobenzene which is commercially availably only as an impure mixture.

The present invention also provides a process for the preparation of a fluoroolefin compound of formula I 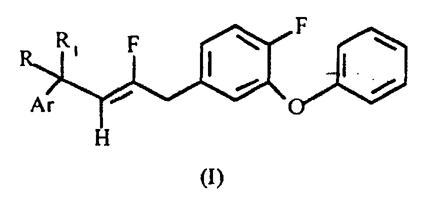 wherein
- Ar is: phenyl optionally substituted with any combination of from one to three halogen, C₁-C₄alkyl, C₁-C₄haloalkyl, C₁-C₄alkoxy or C₁-C₄haloalkoxy groups, or
1- or 2-naphthyl optionally substituted with any combination of from one to three halogen, C₁-C₄alkyl, C₁-C₄haloalkyl, C₁-C₄alkoxy or C₁-C₄haloalkoxy groups;
- R is: hydrogen and R₁ is cyclopropyl, or R and R₁ are each independently C₁-C₄alkyl, or R and R₁ are taken together with the carbon atom to which they are attached to form a cyclopropyl group,
from 5-bromo-2-fluorobenzeneboronic acid.

The process comprises lithiating 1-bromo-4-fluorobenzene, which is conveniently carried out with a lithium base in the presence of a solvent, to form (5-bromo-2-fluorophenyl)lithium, reacting (5-bromo-2-fluorophenyl)lithium with a tri(C₁-C₆alkyl) borate to form a di(C₁-C₆alkyl) 5-bromo-2-fluorobenzeneboronate, hydrolyzing the di(C₁-C₆alkyl) 5-bromo-2-fluorobenzene boronate to form 5-bromo-2-fluorobenzeneboronic acid, oxidizing 5-bromo-2-fluorobenzeneboronic acid to form 5-bromo-2-fluorophenol, reacting 5-bromo-2-fluorophenol with bromobenzene and a base such as sodium hydride to form 5-bromo-2-fluorophenyl ether, reacting 5-bromo-2-fluorophenyl ether with magnesium, and reacting the resulting compound in the presence of a transition metal catalyst such as a cuprous halide, cuprous cyanide or Li₂ CuCl₄ to form the desired fluoroolefin of formula I. The fluoroolefin compound is useful in a pesticide composition.

Compounds of Formula I may have a configuration of the hydrogen atom and the fluorine atom about the double bond which is mutually trans or which is mutually cis. Compounds in which the configuration is mutually trans are preferred. Compounds of Formula I with a trans configuration are prepared using reactant III (as described below) having trans configuration. In order to prepare compounds of Formula I with a cis configuration reactant III having cis configuration is used. In order to prepare compounds of Formula I having a mixture of trans and cis configuration reactant III having a mixture of trans and cis configuration is used.

The invention is described in the following specific embodiments:
1. A process for preparing 5-bromo-2-fluorobenzeneboronic acid, the process comprises lithiating 1-bromo-4-fluorobenzene to form (5-bromo-2-fluorophenyl)lithium; reacting (5-bromo-2-fluorophenyl)lithium with a tri(C₁-C₆alkyl) borate to form a di(C₁-C₆alkyl) 5-bromo-2-fluorobenzeneboronate; and hydrolyzing the di(C₁-C₆alkyl) 5-bromo-2-fluorobenzeneboronate.
2. The process of embodiment 1 wherein the lithiating step is at a temperature of less than about 0 °C.
3. The process of embodiment 2 wherein the temperature is less than about -40 °C.
4. The process of embodiment 1 wherein the 1-bromo-4-fluorobenzene in the lithiating step is reacted with a lithium base.
5. The process of embodiment 4 wherein the lithium base is a lithium dialkylamide or a lithium cyclic amide.
6. The process of embodiment 4 wherein the 1-bromo-4-fluorobenzene in the lithiating step is reacted with the base in the presence of a solvent.
7. The process of embodiment 6 wherein the solvent is an ether.
8. The process of embodiment 1 wherein the tri(C₁-C₆alkyl) borate is trimethyl borate.
9. The process of embodiment 1 wherein the di(C₁-C₆alkyl) 5-bromo-2-fluorobenzeneboronate is hydrolyzed with an organic or mineral acid.
10. The process of embodiment 9 wherein the organic or mineral acid is an aqueous acid.
11. A process for the preparation 5-bromo-2-fluorobenzeneboronic acid which comprises lithiating 1-bromo-4-fluorobenzene with a lithium base in the presence of a solvent to form (5-bromo-2-fluorophenyl)lithium, reacting (5-bromo-2-fluorophenyl)lithium with tri(C₁-C₆alkyl) borate to form a di(C₁-C₆alkyl) 5-bromo-2-fluorobenzeneboronate, and hydrolyzing the di(C₁-C₆alkyl) 5-bromo-2-fluorobenzeneboronate.
12. The process according to embodiment 11 wherein 1-bromo-4-fluorobenzene is lithiated with the lithium base at a temperature below about 0 °C.
13. The process according to embodiment 12 wherein the temperature is below about -40 °C.
14. The process according to embodiment 11 wherein the lithium base is a lithium dialkylamide or a lithium cyclic amide.
15. The process according to embodiment 14 wherein the lithium base is lithium diisopropyl amide.
16. The process according to embodiment 11 wherein the solvent is an ether.
17. The process according to embodiment 16 wherein the ether is tetrahydrofuran.
18. The process according to embodiment 11 wherein the tri(C₁-C₆alkyl) borate is trimethyl borate.
19. The process according to embodiment 11 wherein the di(C₁-C₆alkyl) 5-bromo-2-fluorobenzeneboronate is hydrolyzed with an aqueous organic acid or an aqueous mineral acid.
20. A process for the preparation 5-bromo-2-fluorobenzeneboronic acid which comprises lithiating 1-bromo-4-fluorobenzene with a lithium base selected from the group consisting of a lithium dialkylamide and a lithium cyclic amide in the presence of an ether to form (5-bromo-2-fluorophenyl)lithium with a tri(C₁-C₆alkyl) borate to form a di(C₁-C₆alkyl) 5-bromo-2-fluorobenzeneboronate, and hydrolyzing the di(C₁-C₆alkyl) 5-bromo-2-fluorobenzeneboronate with an aqueous organic or mineral acid.
21. A process for preparing a fluoroolefin compound having the formula 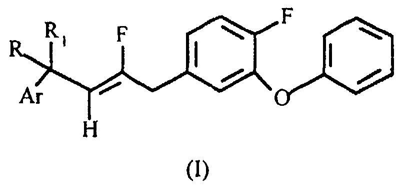 wherein Ar is phenyl optionally substituted with any combination of from one to three halogen, C₁ -C₄alkyl, C₁-C₄haloalkyl, C₁-C₄alkoxy or C₁-C₄haloalkoxy groups, or 1- or 2-naphthyl optionally substituted with any combination of from one to three halogen, C₁ -C₄alkyl, C₁-C₄haloalkyl, C₁-C₄alkoxy or C₁-C₄haloalkoxy groups; R is hydrogen and R₁ is cyclopropyl, or R and R1 are each independently C₁-C₄alkyl, or R and R₁ are taken together with the carbon atom to which they are attached to form a cyclopropyl group; and
   the configuration of the hydrogen atom and the fluorine atom about the double bond is mutually trans which process comprises
   lithiating 1-bromo-4-fluorobenzene with a lithium base in the presence of a solvent to form (5-bromo-2-fluorophenyl)lithium,
   reacting (5-bromo-2-fluorophenyl)lithium with a tri(C₁-C₆alkyl) borate to form a di(C₁ -C₆alkyl) 5-bromo-2-fluorobenzeneboronate,
   hydrolyzing the di(C₁-C₆alkyl) 5-bromo-2-fluorobenzeneboronate to form 5-bromo-2-fluorobenzeneboronic acid,
   oxidizing 5-bromo-2-fluorobenzeneboronic acid to form 5-bromo-2-fluorophenol,
   reacting 5-bromo-2-fluorophenol with bromobenzene and a base to form 5-bromo-2-fluorophenyl ether,
   reacting 5-bromo-2-fluorophenyl phenyl ether with magnesium to form the corresponding magnesium bromide, and
   reacting the magnesium bromide with a compound having the formula 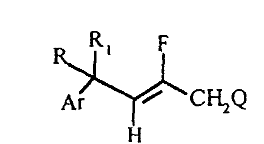 wherein Ar, R and R₁ are as described above and Q is OC(O)CH₃ or Br in the presence of a transition metal catalyst.
22. The process of embodiment 21 wherein the base in the second reacting step is sodium hydride.

The process preferably comprises lithiating 1-bromo-4-fluorobenzene with at least about one molar equivalent of a lithium base in the presence of a solvent preferably at a temperature below about 0 °C, more preferably below about -40 °C, to form (5-bromo-2-fluorophenyl)lithium, reacting (5-bromo-2-fluorophenyl)lithium with at least about one molar equivalent of a tri(C₁-C₆alkyl) borate to form a di(C₁-C₆alkyl) 5-bromo-2-fluorobenzeneboronate, and hydrolyzing the di(C₁-C₆alkyl) 5-bromo-2-fluorobenzene boronate with at least about two molar equivalents of an aqueous acid to form the desired 5-bromo-2-fluorobenzene-boronic acid. The reaction scheme is shown in Flow Diagram I.

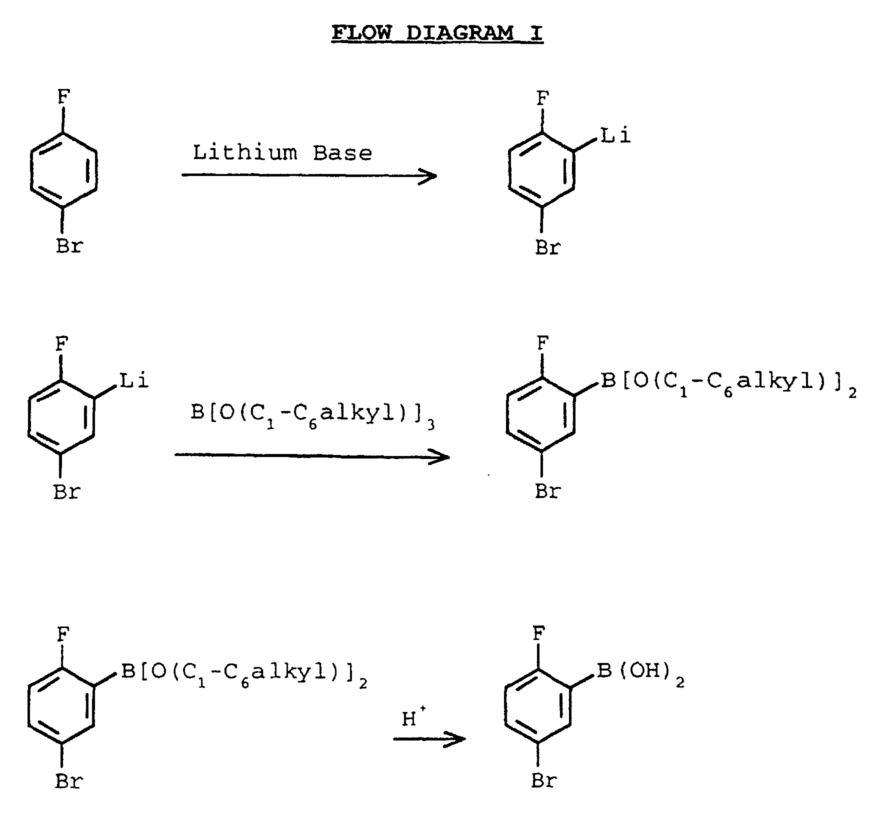

Advantageously, the process of this invention overcomes the problems associated with the use of impure 2,4-dibromofluorobenzene by using 1-bromo-4-fluorobenzene. By avoiding the use of impure 2,4-dibromofluorobenzene, the process of this invention provides 5-bromo-2-fluorobenzeneboronic acid in higher yield and higher purity than the less effective and less efficient art processes.

Lithium bases suitable for use in the process of this invention include lithium secondary amide bases such as lithium dialkylamides, lithium cyclic amides, lithium arylalkylamides and lithium bis(alkylsilyl)amides and alkyl lithiums such as n-butyl lithium, s-butyl lithium, and tert-butyl lithium. Preferred lithium bases include lithium dialkylamides such as lithium diisopropylamide and lithium isopropylcyclohexylamide, lithium cyclic amides such as lithium 2,2,6,6-tetramethylpiperidine, lithium arylalkylamides such as lithium phenylmethylamide, and bis(alkylsilyl)amides such as lithium bis(trimethylsilyl)amide, with lithium diisopropylamide and lithium 2,2,6,6-tetramethylpiperidine being more preferred.

Solvents suitable for use in the process of the present invention include organic solvents which do not react undesirably with any of the compounds present in the reaction mixture. Preferred organic solvents include ethers such as tetrahydrofuran, diethyl ether, 1,2-dimethoxyethane, and mixtures thereof, with tetrahydrofuran being more preferred.

Preferred tri(C₁-C₆alkyl) borates include trimethyl borate, triethyl borate, tri-n-butyl borate and triisopropyl borate with trimethyl borate being more preferred.

The di(C₁-C₆alkyl) 5-bromo-2-fluorobenzeneboronate compound is preferably hydrolyzed with an aqueous organic acid such as acetic acid, propionic acid and butyric acid or an aqueous mineral acid such as hydrochloric acid and sulfuric acid.

Advantageously, the present invention also provides a process for the preparation of a fluoroolefin compound of formula I 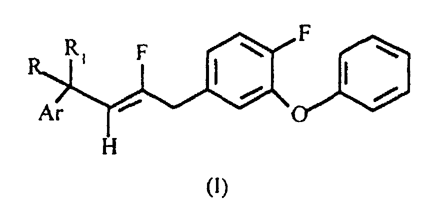 wherein Ar is phenyl optionally substituted with any combination of from one to three halogen, C₁-C₄alkyl, C₁-C₄haloalkyl, C₁-C₄alkoxy or C₁-C₄haloalkoxy groups, or 1- or 2-naphthyl optionally substituted with any combination of from one to three halogen, C₁-C₄alkyl, C₁-C₄haloalkyl, C₁-C₄alkoxy or C₁-C₄haloalkoxy groups; R is hydrogen and R₁ is cyclopropyl, or R and R₁ are each independently C₁-C₄alkyl, or R and R₁ are taken together with the carbon atom to which they are attached to form a cyclopropyl group from 5-bromo-2-fluorobenzeneboronic acid. Compounds of Formula I may have a configuration of the hydrogen atom and the fluorine atom about the double bond which is mutually trans or which is mutually cis.

The process comprises lithiating 1-bromo-4-fluorobenzene, e.g. with a lithium base in the presence of a solvent, to form (5-bromo-2-fluorophenyl)lithium, reacting (5-bromo-2-fluorophenyl)lithium with a tri(C₁-C₆alkyl) borate to form a di(C₁-C₆alkyl) 5-bromo-2-fluorobenzeneboronate, hydrolyzing the di(C₁-C₆alkyl) 5-bromo-2-fluorobenzeneboronate to form 5-bromo-2-fluorobenzeneboronic acid, oxidizing 5-bromo-2-fluorobenzeneboronic acid to form 5-bromo-2-fluorophenol, reacting 5-bromo-2-fluorophenol with bromobenzene and a base such as a sodium hydride to form 5-bromo-2-fluorophenyl ether, reacting 5-bromo-2-fluorophenyl phenyl ether with magnesium to form a magnesium bromide of formula II, and reacting the formula II compound with an alkene compound of formula III in the presence of a transition metal catalyst sych as cuprous halide, cuprous cyanide or Li₂CuCl₄ to form the desired fluoroolefin of formula I. The fluoroolefin compound is useful in a pesticide composition.

The reaction scheme for the preparation of the fluoroolefin compound is shown in Flow Diagram II.

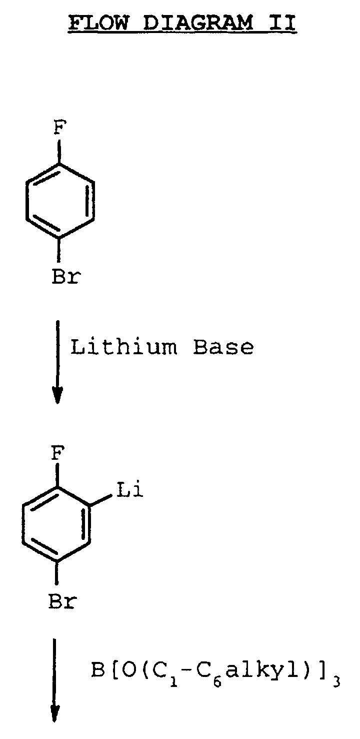

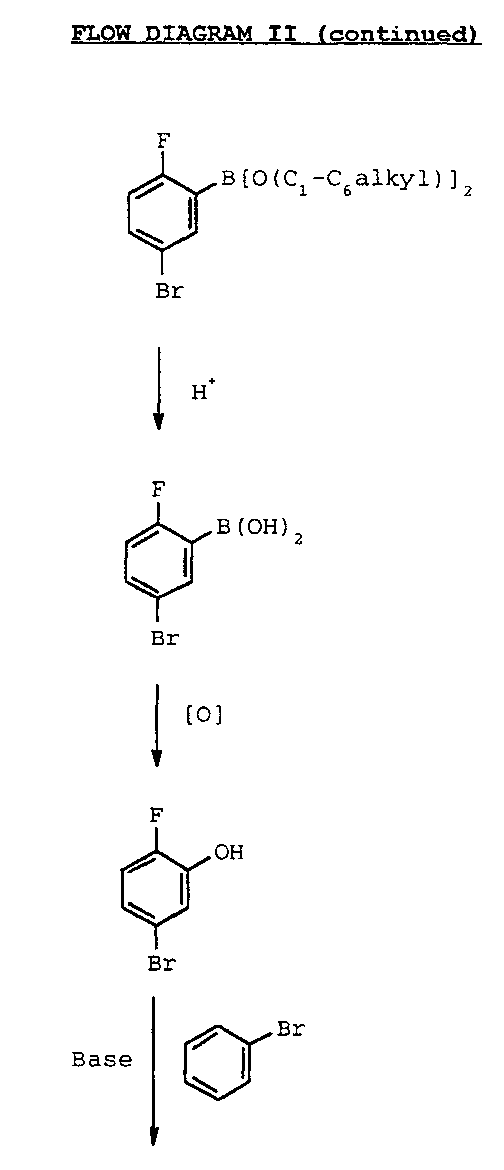

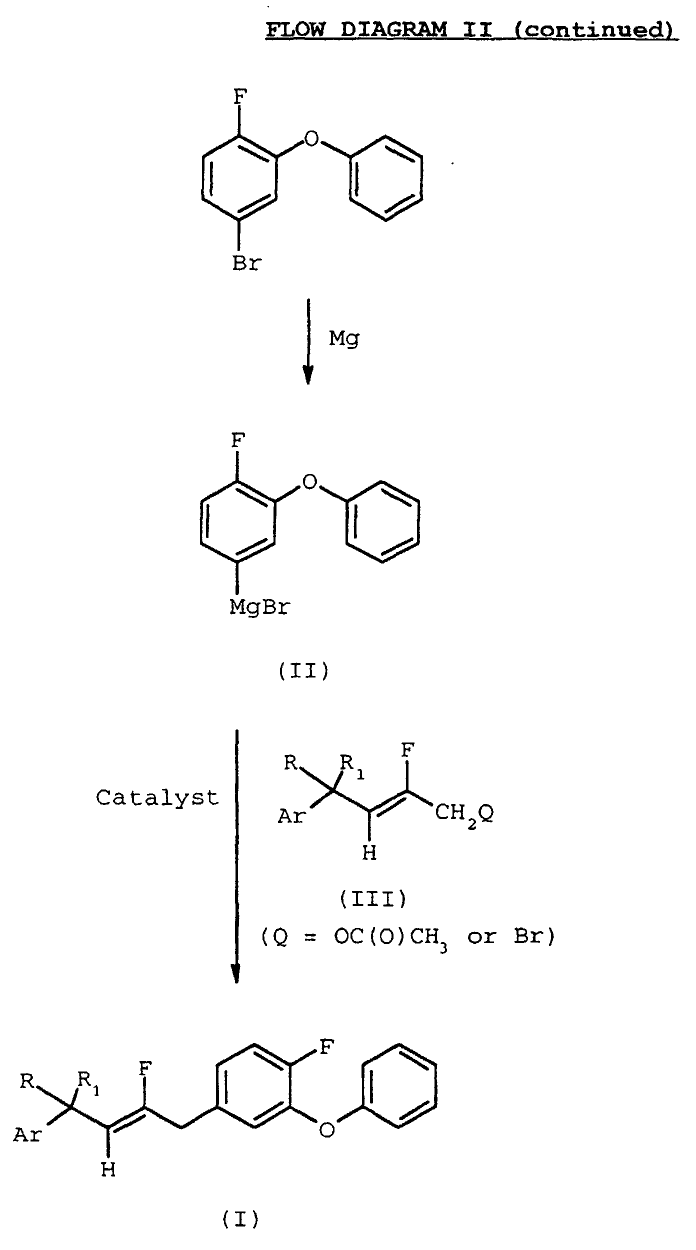

In order to facilitate a further understanding of the invention, the following example is presented to illustrate more specific details thereof. The invention is not to be limited thereby except as defined in the claims.

### EXAMPLE 1

### Preparation of 5 -Bromo -2-fluorobenzeneboronic acid

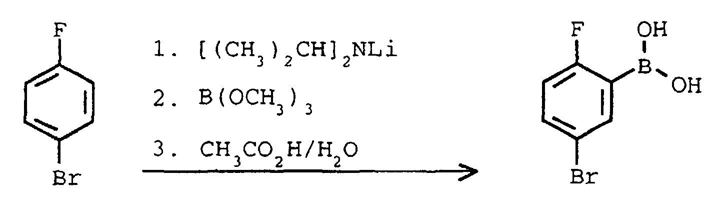

A solution of lithium diisopropylamide (165 mL of a 2.0 M solution in tetrahydrofuran, 0.33 mol) in tetrahydrofuran (600 mL) at -70 °C is treated with 1-bromo-4-fluorobenzene (33.0 mL, 0.30 mol), stirred at -70 °C for 90 minutes and added to a solution of trimethyl borate (41.0 mL, 0.36 mol) in diethyl ether (300 mL) at -70 °C. The resulting solution is stirred at -70 °C for 15 minutes, warmed to 15 °C over 90 minutes, treated with acetic acid (51.5 mL, 0.9 mol) and water (375 mL), and stirred at room temperature for 30 minutes. The organic layer is separated and the aqueous layer is extracted with ether. The organic extracts are combined with the organic layer and the resulting solution is washed sequentially with 10% hydrochloric acid and brine, dried over anhydrous magnesium sulfate and concentrated *in vacuo* to give the title product as an off-white solid (65 g, 99% yield).

## Claims

1. A process for preparing 5-bromo-2-fluorobenzeneboronic acid, the process comprises lithiating 1-bromo-4-fluorobenzene to form (5-bromo-2-fluorophenyl) lithium; reacting (5-bromo-2-fluorophenyl)lithium with a tri (C₁-C₆ alkyl) borate to form a di(C₁-C₆) alkyl 5-bromo-2-fluorobenzeneboronate; and hydrolyzing the di(C₁-C₆alkyl) 5-bromo-2-fluorobenzeneboronate.

2. The process of claim 1 wherein the lithiating step is at a temperature of less than about 0 °C.

3. The process of claim 1 wherein the 1-bromo-4-fluorobenzene in the lithiating step is reacted with a lithium base in the presence of a solvent.

4. The process of claim 1 wherein the tri(C₁-C₆alkyl) borate is selected from the group consisting of trimethyl borate, triethyl borate, tri-n-butyl borate and triisopropyl borate.

5. The process according to claim 2 wherein the temperature is below about -40 °C.

6. The process according to claim 3 wherein the lithium base is a lithium dialkylamide or a lithium cyclic amide.

7. The process according to claim 6 wherein the lithium base is lithium diisopropyl amide.

8. The process according to claim 3 wherein the solvent is an ether.

9. The process according to claim 8 wherein the ether is tetrahydrofuran.

10. The process according to claim 4 wherein the tri(C₁-C₆alkyl) borate is trimethyl borate.

11. The process according to claim 1 wherein the di(C₁-C₆alkyl) 5-bromo-2-fluorobenzeneboronate is hydrolyzed with an aqueous organic acid or an aqueous mineral acid.

12. The process according to claim 1 **characterized by** lithiating 1-bromo-4-fluorobenzene with a lithium base selected from the group consisting of a lithium dialkylamide and a lithium cyclic amide in the presence of an ether to form (5-bromo-2-fluorophenyl)lithium with a tri(C₁-C₆alkyl) borate to form a di(C₁-C₆alkyl) 5-bromo-2-fluorobenzeneboronate, and hydrolyzing the di(C₁-C₆alkyl) 5-bromo-2-fluorobenzeneboronate with an aqueous organic or mineral acid.

13. A process for preparing a fluoroolefin compound having the formula 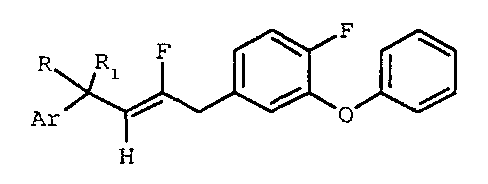 wherein
Ar is phenyl optionally substituted with any combination of from one to three halogen, C₁-C₄alkyl, C₁-C₄haloalkyl, C₁-C₄alkoxy or C₁-C₄haloalkoxy groups, or
1- or 2-naphthyl optionally substituted with any combination of from one to three halogen, C₁-C₄alkyl, C₁-C₄haloalkyl, C₁-C₄alkoxy or C₁-C₄haloalkoxy groups;
R is hydrogen and R₁ is cyclopropyl, or R and R₁ are each independently C₁-C₄alkyl, or R and R₁ are taken together with the carbon atom to which they are attached to form a cyclopropyl group
which process comprises
lithiating 1-bromo-4-fluorobenzene with a lithium base in the presence of a solvent to form (5-bromo-2-fluorophenyl)lithium,
reacting (5-bromo-2-fluorophenyl)lithium with a tri(C₁-C₆alkyl) borate to form a di(C₁-C₆alkyl) 5-bromo-2-fluorobenzeneboronate,
hydrolyzing the di(C₁-C₆alkyl) 5-bromo-2-fluorobenzeneboronate to form 5-bromo-2-fluorobenzeneboronic acid,
oxidizing the 5-bromo-2-fluorobenzeneboronic acid to form 5-bromo-2-fluorophenol,
reacting 5-bromo-2-fluorophenol with bromobenzene and a base to form 5-bromo-2-fluorophenyl phenyl ether,
reacting 5-bromo-2-fluorophenyl phenyl ether with magnesium to form the corresponding magnesium bromide, and
reacting the magnesium bromide with a compound having the structural formula 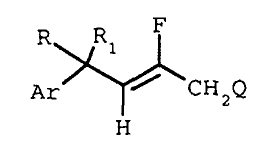 wherein Ar, R and R₁ are as described above and Q is OC(O)CH₃ or Br in the presence of a transition metal catalyst.

14. The process of claim 13 wherein the base in the second reacting step is sodium hydride.

## Patentansprüche

1. Verfahren zur Herstellung von 5-Brom-2-fluorbenzolboronsäure, bei dem man 1-Brom-4-fluorbenzol zu (5-Brom-2-fluorphenyl)lithium lithiiert, (5-Brom-2-fluorphenyl)lithium mit einem Tri(C₁-C₆-alkyl)borat zu einem 5-Brom-2-fluorbenzolboronsäure-di(C₁-C₆-alkyl)ester umsetzt und den 5-Brom-2-fluorbenzolboronsäure-di(C₁-C₆-alkyl)ester hydrolysiert.

2. Verfahren nach Anspruch 1, wobei man den Lithiierungsschritt bei einer Temperatur von unter etwa 0°C durchführt.

3. Verfahren nach Anspruch 1, bei dem man das 1-Brom-4-fluorbenzol im Lithiierungsschritt mit einer Lithiumbase in Gegenwart eines Lösungsmittels umsetzt.

4. Verfahren nach Anspruch 1, bei dem man das Tri(C₁-C₆-alkyl)borat aus der aus Trimethylborat, Triethylborat, Tri-n-butylborat und Triisopropylborat bestehenden Gruppe auswählt.

5. Verfahren nach Anspruch 2, bei dem die Temperatur unter etwa -40°C liegt.

6. Verfahren nach Anspruch 3, wobei es sich bei der Lithiumbase um ein Lithiumdialkylamid oder ein cyclisches Lithiumamid handelt.

7. Verfahren nach Anspruch -6, wobei es sich bei der Lithiumbase um Lithiumdiisopropylamid handelt.

8. Verfahren nach Anspruch 3, wobei es sich bei dem Lösungsmittel um einen Ether handelt.

9. Verfahren nach Anspruch 8, wobei es sich bei dem Ether um Tetrahydrofuran handelt.

10. Verfahren nach Anspruch 4, wobei es sich bei dem Tri(C₁-C₆-alkyl)borat. um Trimethylborat handelt.

11. Verfahren nach Anspruch 1, wobei man den 5-Brom-2-fluorbenzolboronsäure-di(C₁-C₆-alkyl)ester mit einer wäßrigen organischen Säure oder einer wäßrigen Mineralsäure hyrolysiert.

12. Verfahren nach Anspruch 1, **dadurch gekennzeichnet, daß** man 1-Brom-4-fluorbenzol mit einer Lithiumbase ausgewählt aus der aus einem Lithiumdialkylamid und einem cyclischen Lithiumamid bestehenden Gruppe in Gegenwart eines Ethers zu (5-Brom-2-fluorphenyl)lithium lithiiert, (5-Brom-2-fluorphenyl)lithium mit einem Tri(C₁-C₆-alkyl)borat zu einem 5-Brom-2-fluorbenzolboronsäure-di(C₁-C₆-alkyl)ester umsetzt und den 5-Brom-2-fluorbenzolboronsäure-di(C₁-C₆-alkyl)ester mit einer wäßrigen organischen Säure oder einer wäßrigen Mineralsäure hydrolysiert.

13. Verfahren zur Darstellung einer Fluorolefinverbindung mit der Formel 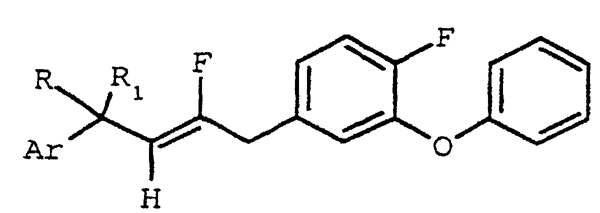 in welcher
Ar für Phenyl steht, das gegebenenfalls durch eine beliebige Kombination aus einer bis drei Halogen-, C₁-C₄-Alkyl-, C₁-C₄-Halogenalkyl-, C₁-C₄-Alkoxyoder C₁-C₄-Halogenalkoxygruppen substituiert ist, oder
für 1- oder 2-Naphthyl steht, das gegebenenfalls durch eine beliebige Kombination aus einer bis drei Halogen-, C₁-C₄-Alkyl-, C₁-C₄-Halogenalkyl-, C₁-C₄-Alkoxy- oder C₁-C₄-Halogenalkoxygruppen substituiert ist;
R für Wasserstoff und R₁ für Cyclopropyl steht, oder R und R₁ jeweils unabhängig voneinander für C₁-C₄-Alkyl stehen, oder R und R₁ zusammen mit dem Kohlenstoffatom, an das sie gebunden sind, eine Cyclopropylgruppe bilden,
bei dem man
1-Brom-4-fluorbenzol mit einer Lithiumbase in Gegenwart eines Lösungsmittels zu (5-Brom-2-fluorphenyl)lithium lithiiert,
(5-Brom-2-fluorphenyl)lithium mit einem Tri(C₁-C₆-alkyl)borat zu einem 5-Brom-2-fluorbenzolboronsäure-di(C₁-C₆-alkyl)ester umsetzt,
den 5-Brom-2-fluorbenzolboronsäuredi(C₁-C₆-alkyl)ester zu 5-Brom-2-fluorbenzolboronsäure hydrolysiert,
die 5-Brom-2-fluorbenzolboronsäure zu 5-Brom-2-fluorphenol oxidiert,
das 5-Brom-2-fluorphenol mit Brombenzol und einer Base zu 5-Brom-2-fluorphenol-phenyl-ether umsetzt,
den 5-Brom-2-fluorphenol-phenyl-ether mit Magnesium zum entsprechenden Magnesiumbromid umsetzt und
das Magnesiumbromid mit einer Verbindung der Strukturformel 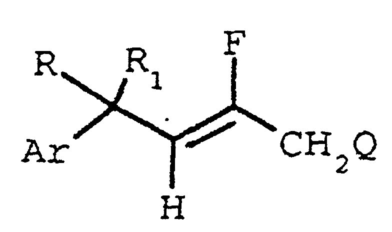 in welcher Ar, R und R₁ wie oben beschrieben sind und Q für OC(O)CH₃ oder Br steht, in Gegenwart eines Übergangsmetallkatalysators umsetzt.

14. Verfahren nach Anspruch 13, wobei es sich bei der Base im zweiten Reaktionsschritt um Natriumhydrid handelt.

## Revendications

1. Procédé de préparation de l'acide 5-bromo-2-fluorobenzèneboronique, le procédé comprenant la lithiation de 1-bromo-4-fluorobenzène pour former le (5-bromo-2-fluorophényl)lithium ; la réaction du (5-bromo-2-fluorophényl)-lithium avec un borate de tri(alkyle en C₃-C₆) pour former un 5-bromo-2-fluorobenzèneboronate de di(alkyle en C₁-C₆) ; et l'hydrolyse du 5-bromo-2-fluorobenzèneboronate de di(alkyle en C₁-C₆).

2. Procédé selon la revendication 1, dans lequel l'étape de lithiation est conduite à une température inférieure à environ 0°C.

3. Procédé selon la revendication 1, dans lequel on fait réagir le 1-bromo-4-fluorobenzène, dans l'étape de lithiation, avec une base lithiée en présence d'un solvant.

4. Procédé selon la revendication 1, dans lequel le borate de tri(alkyle en C₁-C₆) est choisi dans la classe formée par le borate de triméthyle, le borate de triéthyle, le borate de tri-n-butyle et le borate de triisopropyle.

5. Procédé selon la revendication 2, dans lequel la température est inférieure à environ -40°C.

6. Procédé selon la revendication 3, dans lequel la base lithiée est un dialkylamidure de lithium ou un amidure cyclique de lithium.

7. Procédé selon la revendication 6, dans lequel la base lithiée est le diisopropylamidure de lithium.

8. Procédé selon la revendication 3, dans lequel le solvant est un éther.

9. Procédé selon la revendication 8, dans lequel l'éther est le tétrahydrofuranne.

10. Procédé selon la revendication 4, dans lequel le borate de tri(alkyle en C₁-C₆) est le borate de triméthyle.

11. Procédé selon la revendication 1, dans lequel le 5-bromo-2-fluorobenzèneboronate de di(alkyle en C₁-C₆) est hydrolysé avec un acide organique aqueux ou un acide minéral aqueux.

12. Procédé selon la revendication 1, **caractérisé par** la lithiation de 1-bromo-4-fluorobenzène avec une base lithiée choisie dans la classe formée par un dialkylamidure de lithium et un amidure cyclique de lithium en présence d'un éther pour former le (5-bromo-2-fluorophényl)lithium, la réaction du (5-bromo-2-fluorophényl)lithium avec un borate de tri(alkyle en C₁-C₆) pour former un 5-bromo-2-fluorobenzèneboronate de di(alkyle en C₁-C₆), et l'hydrolyse du 5-bromo-2-fluorobenzèneboronate de di(alkyle en C₁-C₆) avec un acide organique ou minéral aqueux.

13. Procédé de préparation d'un composé oléfinique fluoré ayant la formule 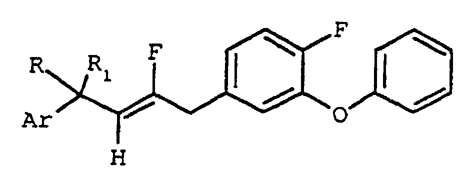 dans laquelle
Ar est un groupe phényle facultativement substitué par toute association de un à trois substituants halogéno, alkyle en C₁-C₄, halogénoalkyle en C₁-C₄, alcoxy en C₁-C₄ ou halogénoalcoxy en C₁-C₄, ou un groupe 1- ou 2-naphtyle facultativement substitué par toute association de un à trois substituants halogéno, alkyle en C₁-C₄, halogénoalkyle en C₁-C₄, alcoxy en C₁-C₄ ou halogénoalcoxy en C₁-C₄ ;
R est l'hydrogène et R₁ est un groupe cyclopropyle, ou bien R et R₁ sont chacun indépendamment un groupe alkyle en C₁-C₄, ou bien R et R₁ sont pris ensemble avec l'atome de carbone auquel ils sont attachés pour former un groupe cyclopropyle
lequel procédé comprend
la lithiation de 1-bromo-4-fluorobenzène avec une base lithiée en présence d'un solvant pour former le (5-bromo-2-fluorophényl)lithium,
la réaction du (5-bromo-2-fluorophényl)lithium avec un borate de tri(alkyle en C₁-C₆) pour former un 5-bromo-2-fluorobenzèneboronate de di(alkyle en C₁-C₆),
l'hydrolyse du 5-bromo-2-fluorobenzèneboronate de di(alkyle en C₁-C₆) pour former l'acide 5-bromo-2-fluorobenzèneboronique,
l'oxydation de l'acide 5-bromo-2-fluorobenzèneboronique pour former le 5-bromo-2-fluorophénol,
la réaction du 5-bromo-2-fluorophénol avec du bromobenzène et une base pour former l'éther de 5-bromo-2-fluorophényle et de phényle,
la réaction de l'éther de 5-bromo-2-fluorophényle et de phényle avec du magnésium pour former le bromure de magnésium correspondant, et
la réaction du bromure de magnésium avec un composé ayant la formule développée 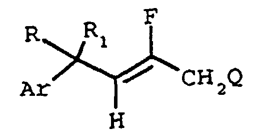 dans laquelle Ar, R et R₁ sont tels que définis ci-dessus et Q est OC(O)CH₃ ou Br, en présence d'un catalyseur à métal de transition.

14. Procédé selon la revendication 13, dans lequel la base, dans la deuxième étape de réaction, est l'hydrure de sodium.
